# EUROPEAN PATENT SPECIFICATION

(11) **EP 2 254 429 B1**
(45) Date of publication and mention of the grant of the patent: **31.10.2018**
(21) Application number: 09721564.4
(22) Date of filing: 16.03.2009
(51) Int. Cl.: A41C 1/02, A41C 1/10, A41D 1/21, A61F 5/03, A61F 13/08

(54) **COMPRESSION GARMENT**
KOMPRESSIONSKLEIDUNG
VÊTEMENT DE COMPRESSION

(30) Priority: 17.03.2008 AU 2008901285; 07.04.2008 AU 2008901644
(43) Date of publication of application: 01.12.2010
(73) Proprietor: Gilheany & O'Donovan Holdings Pty Ltd, Port Melbourne VIC 3207 (AU)
(72) Inventor: DUMPSON, Carmella, Port Melbourne, VIC 3207 (AU); O'DONOVAN, Sinead, Port Melbourne, VIC 3207 (AU)
(74) Representative: Pistolesi, Roberto
(86) International application number: PCT/AU2009/000310
(87) International publication number: WO 2009/114899

(56) References cited:
- WO-A1-2006/032096
- WO-A1-2007/112494
- US-A- 3 301 261
- US-A- 4 343 044
- US-A- 4 625 336
- US-A- 6 062 946
- US-B1- 6 247 185
- US-B2- 6 446 264

## Description

### Introduction

This invention relates to a compression garment and more particularly a compression garment for use in ante and post natal recovery.

### Background of the Invention

Support garments are used both during pregnancy and post pregnancy. US 3301261 A discloses a compressive garment to be used during pregnancy. Most support garments are in the form of underwear or foundation garments. Compression is used for the prevention of deep vein thrombosis and embolism in trauma patients as well as after surgical procedures. Post surgery it has been shown that compression can reduce swelling and provide tissue support which improves the rate and extent of recovery.

Post pregnancy women often have difficulty regaining their pre-pregnancy figure and function. During pregnancy the uterus enlarges to accommodate the developing fetus. The skin over the abdomen along with the abdominal muscles stretch and internal organs displace. After the birth of the baby, the abdomen temporarily remains in an enlarged state and function is impaired. Women often complain that abdominal muscle function never recovers fully and pre pregnancy shape never returns. The surgical procedures (and resultant wounds) required with caesarian deliveries further complicate recovery for women.

During pregnancy, some women suffer from vulval varicosities and pelvic instability. It is known that support garments can help in both reducing the pain and in the treatment of these conditions.

It is the above issues that have brought about the present invention.

### Summary of the Invention

The present invention provides a compression garment according to claim 1. Further embodiments of the invention are provided in the dependent claims. The compression garment is to be worn during or post pregnancy and comprises panels of elasticised fabric joined by seams of flat lock stitching to define, when worn, specific areas of compression, wherein one of the panels extends under the perineum to provide compression to the perineum.

The garment further comprises front and rear leg panels, each leg joined at the inside and outside leg; a waist panel joined to the rear leg panels across the bottom; and a crotch panel extending between ends of the front of the waist panel underneath the perineum to join the rear leg panels, whereby the crotch panel is joined to the top of the front leg panels to maximise compression at the perineum.

In another embodiment the waist panel is replaced by a stomach panel, the ends of which are joined at the middle of the back and the under edge is joined across the top of the rear leg panels and across the top of the crotch panel and front leg panels. In one embodiment the compression garment is in the form of shorts that can be worn as outerwear having a conventional waist line. In another embodiment the compression garment comprises shorts that can be worn as an outer garment with an elevated panel that extends underneath the chest providing upper and lower abdominal support. The joins of the panels or seams are strategically placed to avoid common wound areas and the flat lock seam structure eliminates pressure normally caused by bulky seams.

The positioning of the panels and their joining seams is specifically designed to ensure maximum compression in desired areas. In a preferred embodiment various panels of the garment are lined with an open hole mesh fabric that creates a higher level of compression as well as a thin air barrier or space which promotes dryness and a higher level of moisture vapour transfer. In a preferred
embodiment a yarn such as nylon is mixed with an elasticised yarn such as elastan, the nylon being approximately 80% of the mixture. The preferred weight is 225gsm. In a preferred embodiment the panel fabric is a double knit with jersey stitch face. Where a lining fabric is utilised, it too should have a similar percentage of elastic fabric but is produced by a specific stitch pattern to provide a mini open hole mesh.

In one embodiment, the garment is designed:
- to be an easily utilized over garment;
- to support and compress in all bodily planes which are adversely affected by pregnancy; and
- to support abdominal muscles, caesarian and perineal wounds.

In an embodiment the panels are further designed to provide increased horizontal compression which, in turn, provides consistent compression to varicosities and increased support to reduce the likelihood of pelvic instability.

### Description of the Drawings

Embodiments of the present invention will now be described by way of example only with reference to the accompanying drawings in which:
Figure 1 is a front view of a compression garment illustrating joins between panels of the garment;
Figure 2 is a rear view of the garment;
Figure 3 is a front view of a longer garment;
Figure 4 is a rear view of the longer garment;
Figure 5a is a front view of a modified form of the longer garment shown in Figures 3 and 4;
Figure 5b is a rear view of the garment;
Figures 6a and b are inside front and rear views of the garment;
Figure 7 is a front view of a pregnancy garment;
Figure 8 is a side view of the pregnancy garment; and
Figure 9 is a back view of the pregnancy garment.

### Description of the Preferred Embodiments

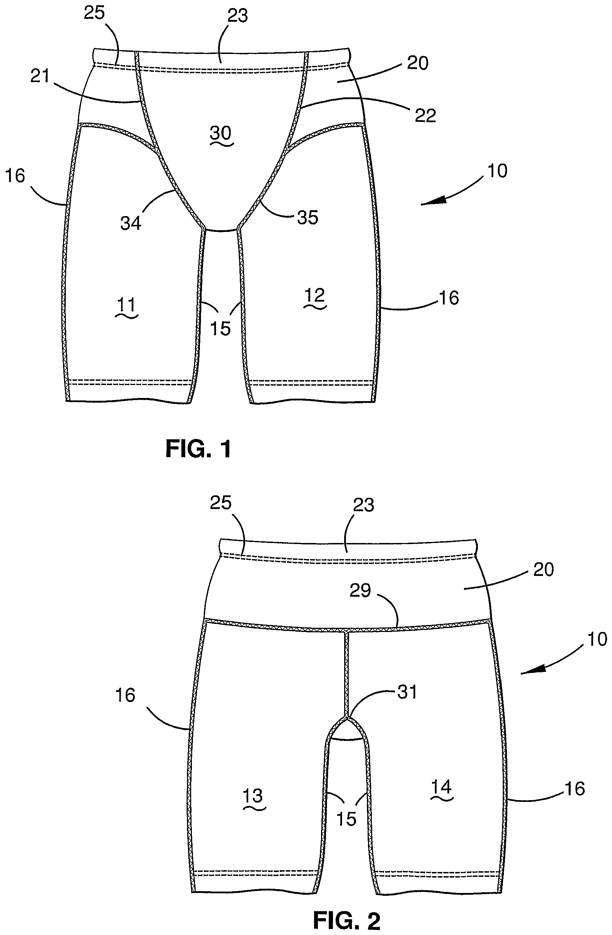

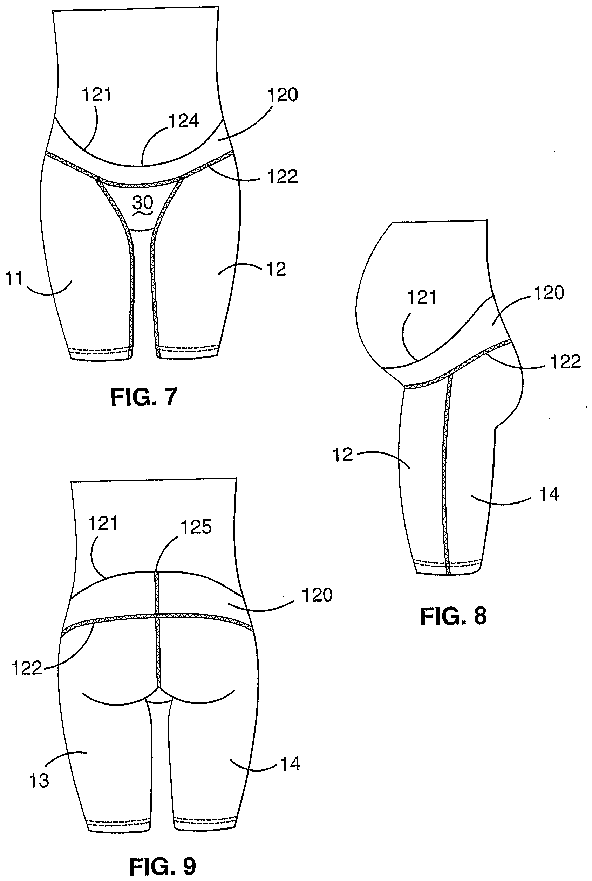

In the embodiments shown in Figures 1 to 6 two styles of compression garment are illustrated specifically for use as outer garments on women post pregnancy. In the embodiment shown in Figures 7 to 9 a compression garment for use as outerwear on women during pregnancy is illustrated.

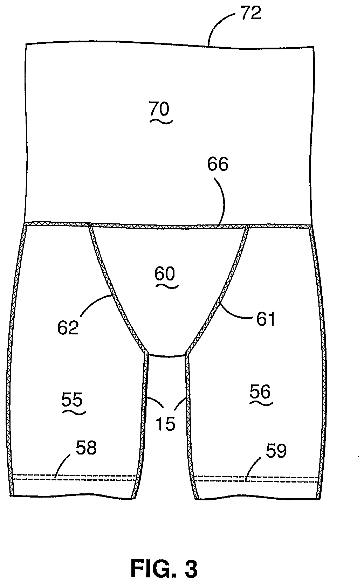

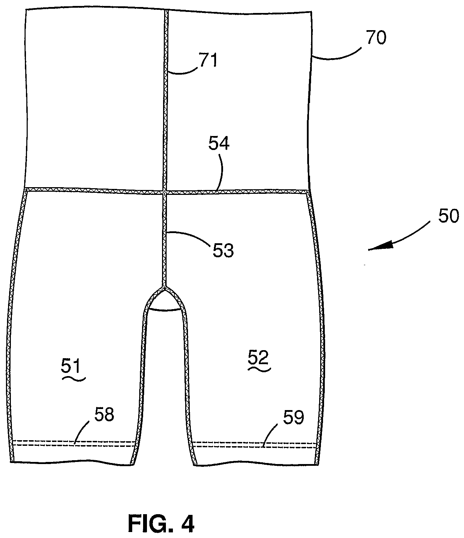

In the first embodiment shown in Figures 1 and 2 a shorter garment 10 is illustrated which sits just below the natural waist line. This garment is more suited to women who have had either caesarian or vaginal delivery. The second embodiment shown in Figures 3 and 4 relates to a higher version 50 of the garment with a wide elastic band 51 sitting just under the bra line. This garment 50 is ideally suited to women who are suffering from rectus abdominus stretching. It also provides compression to the perineum and a caesarian wound.

Both garments 10, 50 have been designed so that they can be worn on the first day post caesarian section or as soon as possible after a vaginal delivery. Preferably the garments 10, 50 are designed as outer garments though it is understood that they could serve the role as undergarments.

Both garments 10, 50 are made of elasticised fabric. Strategically cut panels are stitched together using a flat lock seam stitching to reduce high pressure lines and the likelihood of seams aggravating wounds.

The garment 10 shown in Figures 1 and 2 has front 11, 12 and rear 13, 14 leg panels that are joined at the inner 15 and outer 16 sides of both legs. A waist panel 20 extends across the rear of the garment and is joined along a horizontal seam 29 at the top of the wearer's posterior. The waist panel 20 terminates in ends 21, 22 that are joined to a central crotch panel 30. The crotch panel 30 is heart shaped as shown in Figure 1 and extends under the perineum to join at the base 31 of the posterior at the rear of the garment as shown in Figure 2. The waist panel 20 includes a 1 inch (25.4mm) wide ring of elastic defining a conventional elasticised waist 23. A double needle cover stitched hem 25 finishes the 23 as shown in Figure 1.

As shown in both Figures 1 and 2, the joining of the panels is specifically designed to provide maximum compression to the perineum. Thus, in Figure 1 the front view, the crotch panel 30 is joined to the top of the front leg panels along seams 35, 34 as well as the ends 21, 22 of the waist panel 20. The joining of the crotch panel 30 to the leg panels 11, 12 is specifically provided to enhance the garment to provide maximum compression in the perineum. The crotch panel 30 and waist panel 20 are lined with an open hole mesh panel (not shown) to maximise compression with better breathability / moisture vapour transfer. The crotch panel 30 and the waist panel 20 are single ply shell fabric with a single ply lining. The leg panels 11-14 are single ply unlined. The end of each leg panel 11-14 is hemmed with a double needle coverstitch.

In the embodiment shown in Figures 3 and 4, a longer garment 50 is provided. In this embodiment the garment comprises rear panels 51, 52 for each leg joined at a vertical seam 53 up the rear of the garment to a horizontal cross seam 54 going across the top of the posterior. The front of the garment 50 shown in Figure 3 includes a triangular crotch panel 60 that passes under the perineum and is joined through two inclined seams 61, 62 to the inner upper edges of the front leg panels 55, 56. A horizontal seam 66 runs across the top of the leg panels 55, 56 and crotch panel 60 at the same height as the seam 54 at the rear. A stomach panel 70 comprises a band of fabric that has been doubled and is joined at the rear as shown in Figure 4 by a vertical seam 71. The band 70 extends from the waist to a position just below the chest line. A band of a strip of elastic 72 is encased in the top of the panel 70 to help anchor the panel. The crotch panel 60 is lined with an open hole power mesh to maximise compression for better breathability/moisture and vapour transfer. Double needle coverstitched hems 58, 59 can be placed at the lower ends of each leg portion.

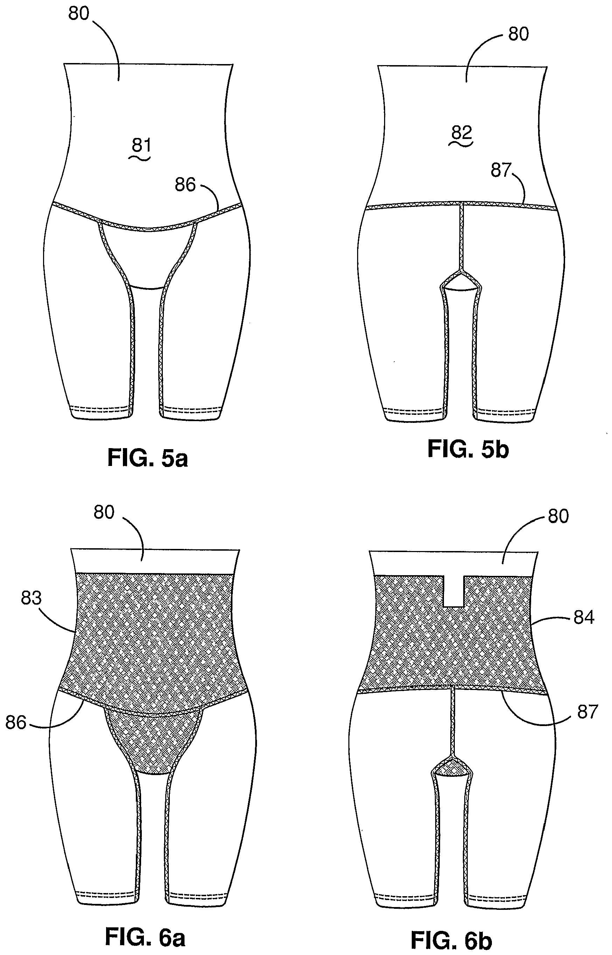

In a third embodiment shown in Figures 5 and 6, a more profiled longer garment is provided in which the stomach panel 80 comprises a contoured outer front panel 81 and contoured back panel 82 joined by side seams. The front and back panels 81, 82 are lined by open hole mesh lining panels 83, 84 which are also contoured. Shaped flat-locked side seams provide better contour and more effective compression of the waist and upper abdomen. The horizontal waist and rear posterior seams 86, 87 are also contoured for more effective compression at the lower abdomen.

In a fourth embodiment shown in Figures 7, 8 and 9, a compression garment is illustrated specifically for use during pregnancy. During the end of the pregnancy term, some women suffer from vulval varicosities which are effectively varicose veins of the vulva. During pregnancy, the valves in the veins do not work as well due to pregnancy hormones which can result in blood pooling in the veins making existing varicose veins worse. Vulval varicosities cause irritation or aching discomfort. Support garments help reduce the irritation and pain.
Pelvic instability is a condition which causes pain around the joins of the pelvis during pregnancy. The hormones associated with pregnancy can cause the joints of the pelvis to loosen which can result in the pelvis becoming unstable or out of alignment. Furthermore, pregnancy places strain on the muscles of the back, stomach, pelvic floor, hips and pelvic girdle which may lead to the pelvic joints becoming less stable. It is thought that support garments help to reduce the effects and pain associated with pelvic instability.

The garment shown in Figures 7 to 9 is specifically constructed to create panels for maximum support of the back and the pelvis and to provide compression of the vulva and labia. This support helps manage pelvic instability and reduce the pain from varicosities. The garment is very similar to the garment shown in Figures 1 and 2 with identical front and rear leg panels 11, 12, 13, 14 and a similar crotch panel 30 but with a modified waist panel 120. In this embodiment, the waist panel 120 is in the form of an ergonomically shaped belt that extends around the torso to maintain consistent contact with the back and sides to create maximum compression for the vulval and labial areas. The belt comprises two outer fabric layers plus an internal lining layer of open hole mesh for additional support and breathability. The waist belt 120 has upper and lower edged 121, 122. The top edge 121 includes a half inch (12.7mm) strip of elastic included in the top edge seam for stability.

The waist belt 120 is narrow as it sits above the symphysis pubis (pubic bone) and then widens outwardly with its maximum width at the lumbar-sacral area at the rear. In a preferred embodiment, the waist band is about 2½ inches (63.5mm) at the narrowest point 124 and increases outwardly to approximately 4½ inches (114.3mm) at the centre of the back 125. The V-shaped crotch panel 30 extends from the front to the rear for horizontal compression of varicosities. The horizontal compression takes place in conjunction with the stabilised belt 120 and leg panels 11-14. The crotch panel 30 and belt 120 are constituted by two outer layers and an internal open hole mesh lining layer. The leg panels 11-14, which finish above the knee, help to maintain placement of the crotch panel 30 to provide consistent compression to varicosities. Each leg panel is a single layer of outer fabric.

Figures 7, 8 and 9 are front, side and rear views and show the contouring and positioning of the waist band across the front, sides and rear of a pregnant torso.

The fabric used in the outer layers of the garments described above is elasticised and preferably comprises 75-85% nylon yarn with 25-15% elastan. The preferred weight is 225gsm. The yarn is preferably nylon and the elasticised material is spandex.

In a preferred embodiment the most appropriate knit stitch to achieve the desired stretch and recovery is to be a jersey stitch face in a double knit construction. However it is also understood that other knitting stitches are envisaged as being suitable.

Recovery should be immediate with no more than +3% variation from the relaxed pre-stretched state to relaxed state immediately after stretch and recovery. The +3% should decrease to 0% variance within 30 minutes. The lining fabric should have a similar content of elastan in an open hole mesh knit stitch pattern. The weight of the lining should be 170gsm.

Whilst these garments have been specifically designed to be worn by women during pregnancy and post delivery, it is understood that these garments may also be used by both male and female patients post surgery. Abdominal surgery such as hernias, appendicitis, liposuction and other plastic surgery provide opportunities for post operative treatment using compressing garments of the kind described above.

The garments provide compression to specific areas and have been designed so that maximum compression is positioned where most needed. It is known that continuous compression on a wound or a swollen area aids recovery by decreasing swelling, improving blood supply thus draining the products of inflammation more effectively. This accelerates the healing process and provides an opportunity for a woman to return to a pre-pregnancy shape more quickly.

Other benefits include reduction and tension of abdominal muscles along with reduced tension over a surgical wound site. The garments provide the patient with core support along with greater ability to return to a daily routine after delivery or surgery. The compression provided by the garment reduces abdominal and perineum tension whilst lifting, bathing and caring for a new born baby.

The sizes of the garments would vary to cover wearers of all sizes.

In the claims which follow and in the preceding description of the invention, except where the context requires otherwise due to express language or necessary implication, the word "comprise" or variations such as "comprises" or "comprising" is used in an inclusive sense, i.e. to specify the presence of the stated features but not to preclude the presence or addition of further features in various embodiments of the invention.

## Claims

1. A compression garment (10; 50) to be worn during or post pregnancy comprising:
front leg panels (11, 12, 55, 56) and rear leg panels (13, 14; 51, 52) joined to form a pair of legs;
a waist panel (20; 120) or a stomach panel (70; 80) attached to a top portion of the pair of legs such that the waist panel (20, 120) or stomach panel (70; 80) in use will at least partially encircle a wearer's body; and
a crotch panel (30; 60) disposed between the front leg panels (11, 12, 55, 56) and extending under the perineum to join the rear leg panels (13, 14; 51, 52);
wherein the front and rear leg panels (11, 12, 13, 14; 51, 52, 55, 56) and the waist panel or the stomach panel (20, 120; 70, 80) of the compression garment (10; 50) are formed from an elasticised fabric and the crotch panel (30; 60) is formed of an elasticised fabric;
**characterised in that**
the crotch panel (30; 60) is joined by flat-lock seams (34, 35; 61, 62) to the front (11, 12, 55, 56) and rear leg panels (13, 14; 51, 52) of the pair of legs, the crotch panel (30; 60) being attached to the waist panel (20; 120) or stomach panel (70; 80) at a front of the compression garment (10; 50) and the flat-lock seams (34, 35; 61, 62) converging towards each other at a rear of the compression garment (10; 50), said flat-lock seams (34, 35; 61, 62) minimizing line pressure at the seams of the compression garment (10; 50);
and wherein the flat-lock seams adjoining the panels (11, 12, 13, 14, 20, 30; 51, 52, 55, 56, 60, 70) are positioned, in use, to avoid common wound areas , and the joining of the panels (11, 12, 13, 14, 20, 30; 51, 52, 55, 56, 60, 70) is configured to apply, in use, maximum compression onto the wearer's perineum.

2. The compression garment (50) according to claim 1, wherein the stomach panel (70; 80) is joined to front leg panels (55, 56) and rear leg panels (51, 52) to form a pair of legs by a horizontal seam (54, 66; 86, 87) across the bottom of the stomach panel (70; 80); and the crotch panel (60) extends from the stomach panel (70; 80) and passes, in use, under the perineum of the wearer to join the rear leg panels (51, 52) at the converging flat-lock seams (61, 62), the crotch panel (60) being joined by frontal lateral flat-lock seams (61, 62) to the top of the front leg panels (55, 56) and by the horizontal seam (66; 86) to the stomach panel (70; 80) to maximize compression onto the wearer's perineum.

3. The compression garment (10; 50) according to claim 1 or 2, wherein the waist panel (20; 120) or stomach panel (70; 80) and the crotch panel (30; 60) comprise a layer of knitted elasticised fabric lined with an open hole mesh elasticised fabric.

4. The compression garment (50) according to any one of claims 1 to 3, wherein the stomach panel (70; 80) is arranged to extend towards a chest line, such that the stomach panel (70; 80), in use, covers and compresses a caesarean wound area and the rectus abdominus muscles of the wearer.

5. The compression garment (10) according to any one of claims 1 or 3, wherein the waist panel (20; 120) is joined to the rear leg panels (13, 14) by a horizontal seam (29; 122) across the bottom of the waist panel (20; 120); and the crotch panel (30) extends downwardly from the waist panel (20; 120) and extends, in use, under the perineum of the wearer to join the rear leg panels (13, 14) at a base (31) where the two rear seams (34, 35)converge at a rear of the garment to maximize compression, in use, onto the wearer's perineum.

6. The compression garment (10; 50) according to any one of claims 1 to 5, wherein
the front (11, 12; 55, 56) and rear (13, 14; 51, 52) leg panels are joined by seams (15, 16) at the inside and outside leg; and the waist panel (20; 120) or the stomach panel (70; 80) is joined to the rear leg panels (13, 14; 51, 52) by a seam (29; 54; 87; 122) across the bottom of the waist panel (20; 120) or the stomach panel (70; 80).

7. The compression garment according to any one of claims 1, 3, 5 or 6, wherein the waist panel (120) is a belt joined by a seam (125) at the back, and being joined to the top of the front leg panels (11, 12) and the top of the rear leg panels (13, 14) and in front, to the top of the crotch panel (30) by shaped seams (122).

8. The compression garment according to claim 7, wherein the belt (120) comprises two outer fabric layers over an internal lining layer of open hole mesh, and the crotch panel (30) comprises two outer layers having an internal lining layer.

9. The compression garment (10; 50) according to any one of claims 1 to 8, wherein the waist panel (20; 120) or stomach panel (70; 80) and the crotch panel (30; 60) comprise at least one layer of elasticised fabric lined with an open hole mesh elasticised fabric.

10. The compression garment (10; 50) according to claim 9, wherein the front (11, 12; 55, 56) and rear (14, 14; 51, 52) leg panels comprise a single layer of elasticised fabric.

11. The compression garment (10; 50) according to any one of the preceding claims, wherein the elasticised fabric comprises a mixture of nylon and elastan.

12. The compression garment (10; 50) according to claim 11, wherein the mixture is 75-85% nylon and 25-15% elastan, preferably 80% nylon and 20% elastan.

13. The compression garment (10; 50) according to any one of the preceding claims, wherein the elasticised fabric comprises a yarn knitted in a double jersey stitch.

14. The compression garment (10; 50) according to any one of the preceding claims, wherein the waist panel (20; 120) or stomach panel (70; 80) comprises elasticised fabric.

15. The compression garment (10; 50) according to any one of the preceding claims, wherein the crotch panel (30; 60) is formed of at least one ply of elasticised fabric.

## Patentansprüche

1. Kompressionskleidung (10; 50), zum Tragen während oder nach der Schwangerschaft, welche umfasst:
Bein-Vorderseiten (11, 12, 55, 56) und Bein-Rückseiten (13, 14; 51, 52), die unter Bildung eines Bein-Paars verbunden sind;
eine Taillen-Seite (20; 120) oder eine Bauch-Seite (70; 80), die an einem oberen Bereich des Bein-Paars angebracht sind, so dass die Taillen-Seite (20, 120) oder die Bauch-Seite (70; 80) bei Verwendung mindestens teilweise den Körper einer Trägerin umgeben; und
eine Schritt-Seite (30; 60), die zwischen den Bein-Vorderseiten (11, 12, 55, 56) vorgesehen ist und sich unter dem Perineum erstreckt, um mit den Bein-Rückseiten (13, 14; 51, 52) verbunden zu sein;
worin die Bein-Vorder- und Rück-Seiten (11, 12, 13, 14; 51, 52, 55, 56) und die Taillen-Seite oder die Bauch-Seite (20, 120; 70, 80) der Kompressionskleidung (10; 50) aus einem dehnfähigen Gewebe ausgebildet sind und die Schritt-Seite (30; 60) aus einem dehnfähigen Gewebe ausgebildet ist;
**dadurch gekennzeichnet, dass**
die Schritt-Seite (30; 60) durch Flatlocknähte (34, 35; 61, 62) mit den und Bein-Vorder-(11, 12, 55, 56) und Rück-Seiten (13, 14; 51, 52) des Bein-Paars verbunden sind, die Schritt-Seite (30; 60) an der Taillen-Seite (20; 120) oder Bauch-Seite (70; 80) an einer Vorderseite der Kompressionskleidung (10; 50) angebracht sind, und die Flatlocknähte (34, 35; 61, 62) an einer Hinterseite der Kompressionskleidung (10; 50) zueinander konvergieren, worin die Flatlocknähte (34, 35; 61, 62) den Liniendruck an den Nähten der Kompressionskleidung (10; 50) minimieren;
und worin die Flatlocknähte neben den Seiten (11, 12, 13, 14, 20, 30; 51, 52, 55, 56, 60, 70) bei Verwendung angeordnet sind, um herkömmliche wunde Bereiche zu vermeiden, und worin die Verbindung der Seiten (11, 12, 13, 14, 20, 30; 51, 52, 55, 56, 60, 70) ausgestaltet ist, bei Verwendung eine maximale Kompression auf das Perineum der Trägerin auszuüben.

2. Kompressionskleidung (50) nach Anspruch 1, worin die Bauch-Seite (70; 80) mit den Bein-Vorderseiten (55, 56) und Bein-Rückseiten (51, 52) verbunden ist, um mittels einer horizontalen Naht (54, 66; 86, 87) über den Boden der Bauch-Seite (70; 80) ein Bein-Paar auszubilden; und worin sich die Schritt-Seite (60) von der Bauch-Seite (70; 80) erstreckt und bei Verwendung unter dem Perineum der Trägerin verläuft, um die Bein-Rückseiten (51, 52) an den konvergierenden Flatlocknähten (61, 62) zu verbinden, worin die Schritt-Seite (60) durch die vorderen seitlichen Flatlocknähten (61, 62) mit der Oberseite der Bein-Vorderseiten (55, 56) verbunden ist und über die horizontale Naht (66; 86) mit der Bauch-Seite (70; 80), um eine Kompression auf das Perineum des Trägers zu maximieren.

3. Kompressionskleidung (10; 50) nach Anspruch 1 oder 2, worin die Taillen-Seite (20; 120) oder Bauch-Seite (70; 80) und die Schritt-Seite (30; 60) eine Schicht aus ein gestricktem dehnfähigem Gewebe umfasst, das mit einem Netz aus dehnfähigem Gewebe mit offenen Löchern ausgekleidet ist.

4. Kompressionskleidung (50) nach einem der Ansprüche 1 bis 3, worin die Bauch-Seite (70; 80) angeordnet ist, sich auf eine Brustlinie zu erstrecken, so dass die Bauch-Seite (70; 80), bei Verwendung, einen Kaiserschnitt-Wundbereich und die Rectus Abdominus Muskeln des Trägers bedecken und komprimieren.

5. Kompressionskleidung (10) nach einem der Ansprüche 1 oder 3, worin die Taillen-Seite (20; 120) mit den Bein-Rückseiten (13, 14) über eine horizontale Naht (29; 122) über den unteren Teil der Taillen-Seite (20; 120) verbunden ist; und worin sich die Schritt-Seite (30) von der Taillen-Seite (20; 120) nach unten erstreckt und sich bei Verwendung unter dem Perineum der Trägerin erstreckt, um die Bein-Rückseiten (13, 14) an einer Basis (31) zu verbinden, an der die beiden rückseitigen Nähte (34, 35) an einer Rückseite des Kleidungsstücks konvergieren, um bei Verwendung die Kompression auf das Perineum der Trägerin zu maximieren.

6. Kompressionskleidung (10; 50) nach einem der Ansprüche 1 bis 5, worin die vorderen (11, 12; 55, 56) und hinteren (13, 14; 51, 52) Beinseiten durch Nähte (15, 16) an der Innenseite und Außenseite des Beins verbunden sind;
und worin die Taillen-Seite (20; 120) oder die Bauch-Seite (70; 80) mit den Bein-Rück-seiten (13, 14; 51, 52) über eine Naht (29; 54; 87; 122) über die Unterseite der Taillen-Seite (20; 120) oder Bauch-Seite (70; 80) verbunden ist.

7. Kompressionskleidung nach einem der Ansprüche 1 , 3, 5 oder 6, worin die Taillen-Seite (120) ein Gürtel ist, der durch eine Naht (125) an der Rückseite befestigt ist, und der mittels geformter Nähte (122) an den oberen Bereich der Bein-Vorderseiten (11, 12) und den oberen Bereich der Bein-Rückseiten (13, 14) und vorne und an den oberen Bereich der Schritt-Seite (30) befestigt ist.

8. Kompressionskleidung nach Anspruch 7, worin der Gürtel (120) über einer inneren Futterschicht des Gewebes mit offenen Löchern zwei äußere Gewebeschichten umfasst, und worin die Schritt-Seite (30) zwei äußere Schichten mit einer inneren Futterschicht umfasst.

9. Kompressionskleidung (10; 50) nach einem der Ansprüche 1 bis 8, worin die Taillen-Seite (20; 120) oder Bauch-Seite (70; 80) und die Schritt-Seite (30; 60) mindestens eine Schicht aus dehnfähigem Gewebe umfasst, das mit einem Netz aus dehnfähigem Gewebe mit offenen Löchern ausgefüttert ist.

10. Kompressionskleidung (10; 50) nach Anspruch 9, worin die vorderen (11, 12; 55, 56) und hinteren (14, 14; 51, 52) Beinseiten eine einzelne Schicht aus dehnfähigem Gewebe umfasst.

11. Kompressionskleidung (10; 50) nach einem der vorstehenden Ansprüche, worin das dehnfähige Gewebe ein Gemisch aus Nylon und Elastan umfasst.

12. Kompressionskleidung (10; 50) nach Anspruch 11, worin das Gemisch aus 75-85% Nylon und 25-15% Elastan, vorzugsweise 80% Nylon und 20% Elastan besteht.

13. Kompressionskleidung (10; 50) nach einem der vorstehenden Ansprüche, worin das dehnfähige Gewebe einem in einer doppelten Jersey-Masche gestrickten Zwirn umfasst.

14. Kompressionskleidung (10; 50) nach einem der vorstehenden Ansprüche, worin die Taillen-Seite (20; 120) oder Bauch-Seite (70; 80) dehnfähiges Gewebe umfasst.

15. Kompressionskleidung (10; 50) nach einem der vorstehenden Ansprüche, worin die Schritt-Seite (30; 60) aus mindestens einer Lage aus dehnfähigem Gewebe gebildet ist.

## Revendications

1. Vêtement de compression (10 ; 50) destiné à être porté pendant ou après la grossesse comprenant :
des panneaux de jambe avant (11, 12, 55, 56) et des panneaux de jambe arrière (13, 14 ; 51, 52) assemblés afin de former une paire de jambes ;
un panneau de ceinture (20 ; 120) ou un panneau d'estomac (70 ; 80) fixé sur une partie supérieure de la paire de jambes de sorte que le panneau de ceinture (20 ; 120) ou le panneau d'estomac (70 ; 80), à l'usage, encercle au moins partiellement le corps de l'utilisatrice ; et
un panneau d'entrejambes (30 ; 60) disposé entre les panneaux de jambe avant (11, 12, 55, 56) et s'étendant sous le périnée afin d'assembler les panneaux de jambe arrière (13, 14 ; 51, 52) ;
dans lequel les panneaux de jambe avant et arrière (11, 12, 13, 14 ; 51, 52, 55, 56) et le panneau de ceinture ou le panneau d'estomac (20, 120 ; 70, 80) du vêtement de compression (10 ; 50) sont formés à partir d'un tissu élastiqué et le panneau d'entrejambes (30 ; 60) est formé avec un tissu élastiqué ;
**caractérisé en ce que** :
le panneau d'entrejambes (30 ; 60) est assemblé par des coutures plates (34, 35 ; 61, 62) sur les panneaux de jambe avant (11, 12, 55, 56) et arrière (13, 14 ; 51, 52) de la paire de jambes, le panneau d'entrejambes (30 ; 60) étant fixé sur le panneau de ceinture (20 ; 120) ou le panneau d'estomac (70 ; 80) à l'avant du vêtement de compression (10 ; 50) et les coutures plates (34, 35 ; 61, 62) convergeant l'une vers l'autre au niveau d'une partie arrière du vêtement de compression (10 ; 50), lesdites coutures plates (34, 35 ; 61, 62) minimisant la pression linéaire sur les coutures du vêtement de compression (10 ; 50) ;
et dans lequel les coutures plates attenantes aux panneaux (11, 12, 13, 14, 20, 30 ; 51, 52, 55, 56, 60, 70) sont positionnées, à l'usage, pour éviter les zones enroulées communes et l'assemblage des panneaux (11, 12, 13, 14, 20, 30 ; 51, 52, 55, 56, 60, 70) est configuré pour appliquer, à l'usage, la compression maximum sur le périnée de l'utilisatrice.

2. Vêtement de compression (50) selon la revendication 1, dans lequel le panneau d'estomac (70 ; 80) est assemblé aux panneaux de jambe avant (55, 56) et aux panneaux de jambe arrière (51, 52) afin de former une paire de jambes par une couture horizontale (54, 66 ; 86, 87) d'un côté à l'autre du fond du panneau d'estomac (70 ; 80) ; et le panneau d'entrejambes (60) s'étend à partir du panneau d'estomac (70, 80) et passe, à l'usage, sous le périnée de l'utilisatrice afin d'assembler les panneaux de jambe arrière (51, 52) au niveau des coutures plates convergentes (61, 62), le panneau d'entrejambes (60) étant assemblé par des coutures plates latérales frontales (61, 62) sur le dessus des panneaux de jambe avant (55, 56) et par la couture horizontale (66 ; 86) au panneau d'estomac (70 ; 80) afin de maximiser la compression sur le périnée de l'utilisatrice.

3. Vêtement de compression (10 ; 50) selon la revendication 1 ou 2, dans lequel le panneau de ceinture (20 ; 120) ou le panneau d'estomac (70 ; 80) et le panneau d'entrejambes (30 ; 60) comprennent une couche de tissu élastiqué tricoté doublé avec un tissu élastiqué en mailles à trous ouverts.

4. Vêtement de compression (50) selon l'une quelconque des revendications 1 à 3, dans lequel le panneau d'estomac (70 ; 80) est agencé pour s'étendre vers une ligne de poitrine, de sorte que le panneau d'estomac (70 ; 80), à l'usage, couvre et comprime une zone de césarienne et le muscle grand droit de l'abdomen de l'utilisatrice.

5. Vêtement de compression (10) selon l'une quelconque des revendications 1 à 3, dans lequel le panneau de ceinture (20 ; 120) est assemblé aux panneaux de jambe arrière (13, 14) par une couture horizontale (29 ; 122) d'un côté à l'autre du fond du panneau de ceinture (20 ; 120) ; et le panneau d'entrejambes (30) s'étend vers le bas à partir du panneau de ceinture (20 ; 120) et s'étend, à l'usage, sous le périnée de l'utilisatrice afin d'assembler les panneaux de jambe arrière (13, 14) au niveau d'une base (31) où les deux coutures arrière (34, 35) convergent à l'arrière du vêtement afin de maximiser la compression, à l'usage, sur le périnée de l'utilisatrice.

6. Vêtement de compression (10 ; 50) selon l'une quelconque des revendications 1 à 5, dans lequel :
les panneaux de jambe avant (11, 12 ; 55, 56) et arrière (13, 14 ; 51, 52) sont assemblés par des coutures (15, 16) au niveau de l'intérieur et de l'extérieur de la jambe ;
et le panneau de ceinture (20 ; 120) ou le panneau d'estomac (70 ; 80) est assemblé aux panneaux de jambe arrière (13, 14 ; 51, 52) par une couture (29 ; 51 ; 87 ; 122) d'un côté à l'autre du fond du panneau de ceinture (20 ; 120) ou du panneau d'estomac (70 ; 80).

7. Vêtement de compression selon l'une quelconque des revendications 1, 3, 5 ou 6, dans lequel le panneau de ceinture (120) est une ceinture assemblée par une couture (125) à l'arrière, et étant assemblée à la partie supérieure des panneaux de jambe avant (11, 12) et la partie supérieure des panneaux de jambe arrière (13, 14) et à l'avant, à la partie supérieure du panneau d'entrejambes (30) par des coutures formées (122).

8. Vêtement de compression selon la revendication 7, dans lequel la ceinture (120) comprend deux couches de tissu externes sur une couche de doublure interne de mailles à trous ouverts, et le panneau d'entrejambes (30) comprend deux couches externes ayant une couche de doublure interne.

9. Vêtement de compression (10 ; 50) selon l'une quelconque des revendications 1 à 8, dans lequel le panneau de ceinture (20 ; 120) ou le panneau d'estomac (70 ; 80) et le panneau d'entrejambes (30 ; 60) comprennent au moins une couche de tissu élastiqué doublé avec un tissu élastiqué en mailles à trous ouverts.

10. Vêtement de compression (10 ; 50) selon la revendication 9, dans lequel les panneaux de jambe avant (11, 12 ; 55, 56) et arrière (14, 14 ; 51, 52) comprennent une seule couche de tissu élastiqué.

11. Vêtement de compression (10 ; 50) selon l'une quelconque des revendications précédentes, dans lequel le tissu élastiqué comprend un mélange de nylon et d'élasthanne.

12. Vêtement de compression (10 ; 50) selon la revendications 11, dans lequel le mélange est de 75-85% de nylon et 25-15% d'élasthanne, de préférence 80% de nylon et 20% d'élasthanne.

13. Vêtement de compression (10 ; 50) selon l'une quelconque des revendications précédentes, dans lequel le tissu élastiqué comprend un fil tricoté selon une maille jersey à deux fontures.

14. Vêtement de compression (10 ; 50) selon l'une quelconque des revendications précédentes, dans lequel le panneau de ceinture (20 ; 120) ou le panneau d'estomac (70 ; 80) comprend un tissu élastiqué.

15. Vêtement de compression (10 ; 50) selon l'une quelconque des revendications précédentes, dans lequel le panneau d'entrejambes (30 ; 60) est formé avec au moins une épaisseur de tissu élastiqué.
